(19) Europäisches Patentamt
European Patent Office
Office européen des brevets

(11) EP 1 889 540 B1

(12) **EUROPEAN PATENT SPECIFICATION**

(45) Date of publication and mention of the grant of the patent:
**02.11.2011 Bulletin 2011/44**

(21) Application number: **06756816.2**

(22) Date of filing: **31.05.2006**

(51) Int Cl.:
***A01N 43/90*** *(2006.01)*

(86) International application number:
**PCT/JP2006/310883**

(87) International publication number:
**WO 2006/129714 (07.12.2006 Gazette 2006/49)**

(54) **PEST CONTROL AGENT**

SCHÄDLINGSBEKÄMPFUNGSMITTEL

AGENT ANTIPARASITAIRE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.06.2005 JP 2005161019**

(43) Date of publication of application:
**20.02.2008 Bulletin 2008/08**

(60) Divisional application:
**09164961.6 / 2 111 756**

(73) Proprietors:
- **Meiji Seika Pharma Co., Ltd.**
  **Tokyo 104-8002 (JP)**
- **The Kitasato Institute**
  **Tokyo 108-8642 (JP)**

(72) Inventors:
- **GOTO, Kimihiko,**
  **c/o Meiji Seika Kaisha, Ltd.**
  **Yokohama-shi,**
  **Kanagawa-Ken 222-8567 (JP)**
- **HORIKOSHI, Ryo,**
  **c/o Meiji Seika Kaisha, Ltd.**
  **Yokohama-shi,**
  **Kanagawa-Ken 222-8567 (JP)**
- **TSUCHIDA, Mariko,**
  **c/o Meiji Seika Kaisha, Ltd.**
  **Yokohama-shi,**
  **Kanagawa-Ken 2222-8567 (JP)**
- **OYAMA, Kazuhiko,**
  **c/o Meiji Seika Kaisha, Ltd.**
  **Yokohama-shi,**
  **Kanagawa-Ken 222-8567 (JP)**
- **OMURA, Satoshi**
  **Tokyo 1570076 (JP)**
- **TOMODA, Hiroshi**
  **Tokyo 1820035 (JP)**
- **SUNAZUKA, Toshiaki**
  **Funabashi-shi,**
  **Chiba -Ken 273-0043 (JP)**

(74) Representative: **Warcoin, Jacques et al**
**Cabinet Regimbeau**
**20, rue de Chazelles**
**75847 Paris Cedex 17 (FR)**

(56) References cited:

| | |
|---|---|
| **WO-A-94/09147** | **WO-A1-2004/060065** |
| **JP-A- 08 259 569** | **JP-A- 08 269 062** |
| **JP-A- 08 269 065** | **JP-A- 08 269 066** |

- **WANG H-J ET AL: "Aflavinines and other antiinsectan metabolites from the ascostromata of Eupenicillium crustaceum and related species" APPLIED AND ENVIRONMENTAL MICROBIOLOGY, AMERICAN SOCIETY FOR MICROBIOLOGY, US, vol. 61, no. 12, 1 December 1995 (1995-12-01), pages 4429-4435, XP003023151 ISSN: 0099-2240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **OBATA R ET AL: "Chemical Modification and Structure-activity Relationships of Pyripyropenes. 3. Synthetic conversion of pyridine-pyrone moiety" JOURNAL OF ANTIBIOTICS, JAPAN ANTIBIOTICS RESEARCH ASSOCIATION, TOKYO, JP, vol. 50, no. 3, 1 March 1997 (1997-03-01), pages 229-236, XP003023150 ISSN: 0021-8820**
- **TOMODA H ET AL: "PYRIPYPROPENES, NOVEL INHIBITORS OF ACYL-COA:CHOLESTEROL ACYLTRANSFERASE PRODUCED BY ASPERGILLUS FUMIGATUS I. PRODUCTION, ISOLATION, AND BIOLOGICAL PROPERTIES" JOURNAL OF ANTIBIOTICS, JAPAN ANTIBIOTICS RESEARCH ASSOCIATION, TOKYO, JP, vol. 47, no. 2, 1 February 1994 (1994-02-01), pages 148-153, XP009060945 ISSN: 0021-8820**
- **OBATA R. ET AL.: 'Chemical Modification and Structure-activity Relationships of Pyripyropenes. 1. Modification at the Four Hydroxyl Groups' THE JOURNAL OF ANTIBIOTICS vol. 49, no. 11, 1996, pages 1133 - 1148, XP003002774**


Remarks:

The file contains technical information submitted after the application was filed and not included in this specification

## Description

[BACKGROUND OF THE INVENTION]

### Field of Invention

The present invention relates to a composition for use as a pest control agent comprising a pyripyropene derivative as active ingredient.

### Background Art

Pyripyropene A has inhibitory activity against ACAT (acyl-CoA: cholesterol acyltransferase) and is expected to be applied, for example, to the treatment of diseases induced by cholesterol accumulation, as described in Japanese Patent No. 2993767 (Japanese Patent Laid-Open Publication No. 360895/1992) and Journal of Antibiotics (1993), 46 (7), 1168-9.

Further, pyripyropene analogues and derivatives and ACAT inhibitory activity thereof are described in Journal of Society of Synthetic Organic Chemistry, Japan (1998), Vol. 56, No. 6, pp. 478-488, WO 94/09417, Japanese Patent Laid-Open Publication No. 184158/1994, Japanese Patent Laid-Open Publication No. 239385/1996, Japanese Patent Laid-Open Publication No. 259569/1996, Japanese Patent Laid-Open Publication No. 269062/1996, Japanese Patent Laid-Open Publication No. 269063/1996, Japanese Patent Laid-Open Publication No. 269064/1996, Japanese Patent Laid-Open Publication No. 269065/1996, Japanese Patent Laid-open Publication No. 269066/1996, Japanese Patent Laid-Open Publication No. 291164/1996, and Journal of Antibiotics (1997), 50(3), 229-36.

Furthermore, Applied and Environmental Microbiology (1995), 61(12), 4429-35 describes that pyripyropene A has insecticidal activity against larvae of Helicoverpa zea. Furthermore, WO 2004/060065 describes that pyripyropene A has insecticidal activity against Plutella xylostella L larvae and Tenebrio molitor L. In these documents, however, there is no specific description on insecticidal activity of pyripyropene A against other pests.
Further, none of the above documents describes insecticidal activity of pyripyropene analogues and derivatives.

Up to now, many compounds having insecticidal activity have been reported and have been used as pest control agents. However, the presence of insect species, which are resistant to or can be hardly controlled by these compounds, has posed a problem. Accordingly, the development of a novel pest control agent having excellent insectidal activity has still been desired.

[SUMMARY OF THE INVENTION]

The present inventors have found novel pyripyropene derivatives represented by formula (Ib) having significant insecticidal activity.
The present invention has been made based on such finding.
Accordingly, an object of the present invention is to provide a composition useful as a pest control agent, that comprises a pyripyropene derivative having significant insecticidal activity as active ingredient and can reliably exhibit the contemplated effect and can be used safely. Another object of the present invention is to provide a hemipteran pest control agent that comprises pyripyropene A derivative as active ingredient and can reliably exhibit the contemplated effect and can be used safely. A further object of the present invention is to provide a novel pyripyropene derivative having significant insecticidal activity.

The pyripyropene derivative according to the present invention comprises a compound represented by formula (Ib) according to claim 1 or an agriculturally and horticulturally acceptable salt thereof:
wherein
$Het_1$ represents 3-pyridyl,
$R_1$ represents hydroxyl,
$R_2$ and $R_3$ represent cyclopropylcarbonyloxy, and
$R_4$ represents hydroxyl, cyclopropylcarbonyloxy or 2-cyanobenzoyloxy,

The pyripyropene derivatives represented by formula (Ib) according to the present invention have excellent control effect against agricultural and horiticultural pests, sanitary pests, parasites of animals, stored grain pests, clothing pests, and house pests and a compositions comprising the pyripyropene derivatives as active ingredient can be advantageously utilized as a novel pest control agent.

[DETAILED DESCRIPTION OF THE INVENTION]

Compounds of formula (Ib) or its agriculturally and horticulturally acceptable salts

Compounds of formula (Ib) are novel pyripyropene derivatives. In particular, they have significant insecticidal activity.

According to a preferred embodiment of the present invention, there is provided a composition for use as a pest control agent comprising a compound represented by formula (Ib) or an agriculturally and horticulturally acceptable salt thereof as an active ingredient and an agriculturally and horticulturally acceptable carrier.

Agriculturally and horticulturally acceptable salts in the compounds of formula (Ib) include, for example, acid addition salts such as hydrochlorides, nitrates, sulfates, phosphates, or acetates.

Specific examples of the compounds represented by formula (Ib) include compounds shown in Tables 2 or 3 below.

[Table 1]

Table 1

| Comparative example | $R_1$ | $R_2$ | $R_3$ | $R_4$ | $Het_1$ |
|---|---|---|---|---|---|
| 102 | OH | OH | OH | OH | 3-pyridyl |

[Table 2]

Table 2

| Compound No. | $R_1$ | $R_2$ | $R_3$ | $R_4$ | $Het_1$ |
|---|---|---|---|---|---|
| 218 | OH | OCO-c-$C_3H_5$ | OCO-c-$C_3H_5$ | OCO-c-$C_3H_5$ | 3-pyridyl |

[Table 3]

Table 3

| Compound No. | $R_1$ | $R_2$ | $R_3$ | $R_4$ | $Het_1$ |
|---|---|---|---|---|---|
| 261 | OH | OCO-C-$C_3H_5$ | OCO-C-$C_3H_5$ | OH | 3-pyridyl |

Production process

The compositon according to the present invention can be prepared by mixing the compound represented by formula (Ib) as active ingredient with an agriculturally and horticulturally acceptable carrier. The compound represented by formula (Ib) according to the present invention can be produced according to the following procedure.

Among the compounds according to the present invention, the compounds represented by formula (II) can be synthesized by the method described in Japanese Patent Laid-Open Publication No. 259569/1996, Japanese Patent Laid-Open Publication No. 269062/1996, Japanese Patent Laid-Open Publication No. 269065/1996, or Journal of Antibiotics (1997), 50(3), pp. 229-36. When pyripyropene A is used as a starting material, pyripyropene A, produced by the method described in Journal of Society of Synthetic Organic Chemistry, Japan (1998), Vol. 56, No. 6, pp. 478-488 or WO 94/09417, may be used as the starting material.

[Chemical formula 4]

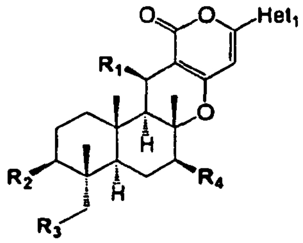

(II)

Wherein $Het_1$ represents 3-pyridyl,
$R_1$ represents hydroxyl, and
$R_2$, $R_3$ and $R_4$ are as defined in formula (Ib).

Use

Insect species against which pyripyropene derivatives of formula (Ib) according to the present invention have control effect include: lepidopteran pests, for example, Spodoptera litura, Mamestra brassicae, Pseudaletia separata, green caterpillar, Plutella xylostella, Spodoptera exigua, Chilo suppressalis, Cnaphalocrocis medinalis, Tortricidae, Carposinldae, Lyonetiidae, Lymantriidae, pests belonging to the genus Agrotis spp., pests belonging to the genus Helicoverpa spp., and pests belonging to the genus Heilothis spp.; hemipteran pests, for example, Aphidoldea including Aphididae, Adelgidae and Phylloxeridae such as Myzus persicae, Aphis - gossypii, Aphis fabae, Aphis maidis (corn-leaf aphid), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, and Toxoptera citricidus; Deltocephalidae such as Nephotettix cincticeps, Delphacidae such as Laodelphax striatellus, Nilaparvata lugens, and Sogatella furcifera; Pentatomidae such as Eysarcoris ventralis, Nezara viridula, and Trigonotylus coelestialium; Aleyrodidae such as Bemisia argentifolii, Bemisia tabaci, and Trialeurodes vaporariorum; Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidae, such as Pseudococcus comstocki and Planococcus citri Risso; Coleoptera pests, for example, Lissorhoptrus oryzophilus, Callosobruchuys chienensis, Tenebrio molitor, Diabrotica virgifera virgifera, Diabrotica undecimpunctata howardi, Anomala cuprea, Anomala rufocuprea, Phyllotreta striolata, Aulacophora femoralis, Leptinotarsa decemlineata, Oulema oryzae, Carposinidae, and Cerambycidae; Acari, for example, Tetranychus urticae, Tetranychus kanzawai, and Panonychus citri; Hymenopteran pests, for example, Tenthredinidae; Orthopteran pests, for example, Acrididae; Dipteran pests, for example, Muscidae and Agromyzidae; Thysanopteran pests, for example, Thrips palmi and Frankliniella occidentalis; Plant Parasitic Nematodes, for example, Meloidogyne hapla, Pratylenchus spp., Aphelenchoides besseyi and Bursaphelenchus xylophilus; and parasites of animals, for example, Siphonaptera, Anoplura, mites such as Boophilus microplus, Haemaphysalis longicornis, Rhipicephalus sanguineus, and Scarcoptes scabiei. Preferred are hemipteran pests.

The composition according to the present invention can be prescribed in any suitable formulation, such as emulsifiable concentrates, liquid formulations, suspension, wettable powder, flowables, dust, granules, tablets, oil solutions, aerosols, or smoking agents by using suitable agriculturally and horticulturally acceptable carriers. Accordingly, the carrier include solid carriers, liquid carriers, gaseous carriers, surfactants, dispersants and/or other adjuvants for formulations, and the like.

Solid carriers usable herein include, for example, talc, bentonite, clay, kaolin, diatomaceous earth, vermiculite, white carbon, and calcium carbonate.

Examples of liquid carriers include: alcohols, such as methanol, n-hexanol, and ethylene glycol; ketones, such as acetone, methyl ethyl ketone, and cyclohexanone; aliphatic hydrocarbons, such as n-hexane, kerosine, and kerosene; aromatic hydrocarbons, such as toluene, xylene, and methylnaphthalene; ethers, such as diethyl ether, dioxane, and tetrahydrofuran; esters, such as ethyl acetate; nitriles, such as acetonitrile and isobutyronitrile; acid amides, such as dimethylformamide and dimethylacetamide; vegetable oils, such as soy bean oil and cotton seed oil; dimethylsulfoxide; and water.

Gaseous carriers include, for example, LPG, air, nitrogen, carbon dioxide, and dimethyl ether.

Surfactants or dispersants usable, for example, for emulsifying, dispersing, or spreading include, for example, alkylsulfonic esters, alkyl(aryl)sulfonic acid salts, polyoxyalkylene alkyl(aryl) ethers, polyhydric alcohol esters, and lignin

sulfonic acid salts.

Adjuvants usable for improving the properties of formulations include, for example, carboxymethylcellulose, gum arabic, polyethylene glycol, and calcium stearate.

The above carriers, surfactants, dispersants, and adjuvant may be used either solely or in combination according to need.

The content of the active ingredient in the formulation is not particularly limited. In general, however, the content of the active ingredient is 1 to 75% by weight for emulsifiable concentrates, 0.3 to 25% by weight for dust, 1 to 90% by weight for wettable powder, and 0.5 to 10% by weight for granules.

The compound represented by formula (Ib), or an agriculturally and horticulturally acceptable salt thereof and the above formualtions comprising the same may be applied as such or after dilution to plants or soil.

According to a further aspect of the present invention, there is provided a method for controlling a pest, comprising applying an effective amount of a compound represented by formula (Ib) or an agriculturally and horticulturally acceptable salt thereof to a plant or soil. Preferred methods usable for applying the compound or formulation to plants or soil include spreading treatment, soil treatment, surface treatment, and fumigation treatment.

Spreading treatments include, for example, spreading, spraying, misting, atomizing, granule application, and submerged application. Soil treatments include, for example, soil affusion and soil mixing, Examples of surface treatments include, for example, coating, dust coating, and covering. Fumigation treatments include, for example, covering of soil with a polyethylene film after soil injection. Accordingly, the control method according to the present invention comprises a method in which the compound represented by formula (Ib) or a formulation comprising the same is applied by fumigation in a sealed space.

The composition according to the present invention may be used as a mixture or in a combination with, for example, other insecticides, fungicides, miticides, herbicides, plant growth-regulating agents, or fertilizers. Agents which may be mixed or used in combination include those described, for example, in The Pesticide Manual, 13th edition, published by The British Crop Protection Council; and SHIBUYA INDEX, the 10th edition, 2005, published by SHIBUYA INDEX RESEARCH GROUP. More specifically, insecticides usable herein include, for example, organophosphate ester compounds such as acephate, dichlorvos, EPN, fenitrothion, fenamifos, prothiofos, profenofos, pyraclofos, chlorpyrifos-methyl, and diazinon; carbamate compounds such as methomyl, thiodicarb, aldicarb, oxamyl, propoxur, carbaryl, fenobucarb, ethiofencarb, fenothiocarb, pirimicarb, carbofuran, and benfuracarb; nereistoxin derivatives such as cartap and thiocyclam; organochlorine compounds such as dicofol and tetradifon; pyrethroid compounds such as permethrin, tefluthrin, cypermethrin, deltamethrin, cyhalothrin, fenvalerate, fluvalinate, ethofenprox, and silafluofen; benzoylurea compounds such as diflubenzuron, teflubenzuron, flufenoxuron, and chlorfluazuron; juvenile hormone-like compounds such as methoprene; and molting hormone-like compounds such as chromafenozide. Other compounds usable herein include buprofezin, hexythiazox, amitraz, chlordimeform, pyridaben, fenpyroxymate, pyrimidifen, tebufenpyrad, fluacrypyrim, acequinocyl, cyflumetofen, flubendiamide, ethiprole, fipronil, ethoxazole, imidacloprid, chlothianidin, pymetrozine, bifenazate, spirodiclofen, spiromesifen, flonicamid, chlorfenapyr, pyriproxyfene, indoxacarb, pyridalyl, or spinosad, avermectin, milbemycin, organometallic compounds, dinitro compounds, organosulfur compounds, urea compounds, triazine compounds, hydrazine compounds.

The composition according to the present invention may also be used as a mixture or in a combination with microbial pesticides such as BT formulations and entomopathogenic viral agents.

Fungicides usable herein include, for example, strobilurin compounds such as azoxystrobin, kresoxym-methyl, and trifloxystrobin; anilinopyrimidine compounds such as mepanipyrim, pyrimethanil, and cyprodinil; azole compounds such as triadimefon, bitertanol; triflumizole, etaconazole, propiconazole, penconazole, flusilazole, myclobutanil, cyproconazole, tebuconazole, hexaconazole, prochloraz, and simeconazole; quinoxaline compounds such as quinomethionate; dithiocarbamate compounds such as maneb, zineb, mancozeb, polycarbamate, and propineb; phenylcarbamate compounds such as diethofencarb; organochlorine compounds such as chlorothalonil and quintozene; benzimidazole compounds such as benomyl, thiophanate-methyl, and carbendazole; phenylamide compounds such as metalaxyl, oxadixyl, ofurace, benalaxyl, furalaxyl, and cyprofuram; sulfenic acid compounds such as dichlofluanid; copper compounds such as copper hydroxide and oxine-copper; isoxazole compounds such as hydroxyisoxazole; organophosphorus compounds such as fosetyl-aluminium and tolclofos-methyl; N-halogenothioalkyl compounds such as captan, captafol, and folpet; dicarboxyimide compounds such as procymidone, iprodione, and vinchlozolin; benzanilide compounds such as flutolanil and mepronil; morpholine comopounds such as fenpropimorph and dimethomorph; organotin compounds such as fenthin hydroxide, and fenthin acetate; and cyanopyrrole compounds such as fludioxonil and fenpiclonil. Other compounds usable herein include fthalide, fluazinam, cymoxanil, triforine, pyrifenox, fenarimol, fenpropidin, pencycuron, cyazofamid, iprovalicarb, and benthiavalicarb-isopropyl and the like.

According to still another aspect of the present invention, there is provided use of a compound represented by formula (Ib) or an agriculturally and horticulturally acceptable salt thereof as a pest control agent.

[EXAMPLES]

The present invention is further illustrated by the following Examples that are not intended as a limitation of the invention. The compound Nos. correspond to the compound Nos. in Tables 1 to 3.

Example 1: Synthesis of compound 218

Compound 102 (30 mg) synthesized by the method described in Japanese Patent Laid-Open Publication No. 259569/1996 and cyclopropanecarboxylic acid (112 mg) were dissolved in anhydrous N,N-dimethylformamide (2 ml), and 1-ethyl-3-(3-dimethylaminopropyl)-carbodiimide hydrochloride (76 mg) and 4-(dimethylamino)pyridine (32 mg) were added to the solution. The reaction solution was stirred at room temperature for 68 hr and was then poured into water, followed by extraction with ethyl acetate. The ethyl acetate layer was washed with saturated brine and was dried over anhydrous magnesium sulfate, and the solvent was removed by evaporation under the reduced pressure to give a crude product of compound 218. The crude product was purified by preparative thin-layer chromatography (Merck Silica Gel 60 $F_{254}$ 0.5mm, acetone : hexane = 1 : 1) to give compound 218 (33 mg).
Mass spectrometric data ($FAB^+$): $662(M+H)^+$

Example 2: Synthesis of compound 261

Compound 218 (1.07 g) prepared in Example 1 was dissolved in an 80% aqueous methanol solution. 1,8-Diazabicyclo[5.4.0]-undeca-7-ene (271 mg) was added to the solution, and the mixture was stirred at room temperature for 24.5 hr. The reaction mixture was added with acetic acid to quench the reaction, and the solvent was removed by evaporation under the reduced pressure. Water was added to the precipitated crystal, followed by extraction with chloroform. The chloroform layer was washed with saturated brine and was dried over anhydrous magnesium sulfate, and the solvent was removed by evaporation under the reduced pressure to give a crude product of compound 261. The crude product was purified by chromatography on silica gel (Mega Bond Elut (Varian), acetone : hexane = 1 : 1) to give compound 261 (233 mg).
Mass spectrometric data ($ESI^+$): $594(M+H)^+$

Example 3: Synthesis of compound 269

Compound 261 (20 mg) prepared in Example 2 and 2-cyanobenzoic acid (30 mg) were dissolved in anhydrous N,N-dimethylformamlde (1 ml), and 1-ethyl-3-(3-dimethylamtnopropyl)-carbodiimide hydrochloride (26 mg) and 4-(dimethylamino)pyridine (4 mg) were added to the solution. The reaction solution was stirred at room temperature for 12 hr, and the reaction solution was added to water, followed by extraction with ethyl acetate. The ethyl acetate layer was washed with saturated brine and was dried over anhydrous magnesium sulfate. The solvent was removed by evaporation under the reduced pressure to give a crude product of compound 269. The crude product was purified by preparative thin-layer chromatography (Merck Silica Gel 60 $F_{254}$ 0.5mm, acetone : hexane = 1 : 1) to give compound 269 (18 mg).
Mass spectrometric data ($ESI^+$): 723 $(M+H)^+$
[Table 4]

Table 4

| Compound No. | $^1$H-NMR δ (ppm) |
|---|---|
| 218 | 0.83-1.12 (12H, m), 0.91 (3H, s), 1.26 (1H, s), 1.33-1,41 (1H, m), 1.45 (3H, s), 1.52-1.69 (6H, m), 1.71 (3H, s), 1.81-1.93 (2H. m), 2.14-2.18 (1H, m), 2.92 (1H, m), 3.72 (1H, d, J = 11.9 Hz), 3.82 (1H, d, J = 11.9 Hz), 4.80 (1H, dd, J = 4.9, 11.4 Hz), 4.99-5.04 (2H, m), 6.46 (1H, s), 7.41 (1H, dd, J = 4.9. 8.3 Hz), 8.10 (1H, dt, J = 1.7, 8.3 Hz), 8.69 (1H, dd, J = 1.5, 4.9 Hz), 9.01 (1H, d, J = 1.4 Hz) |

[Table 5]

Table 5

| Compound | No. $^1$H-NMR δ (ppm) |
|---|---|
| 261 | 0.85-1.08 (8H, m), 0.92 (3H, s), 1.26 (1H, s), 1.30-1.40 (1H, m), 1.42 (3H, s), 1.45-1.63 (5H, m), 1.67 (3H, s), 1.81-1.92 (2H, m), 2.14-225 (2H, m), 2.88 (1H, d, J = 1.4 Hz), 3.75 (1H, d, J = 11.9 Hz), 3.86 (1H, d, J = 11.6 Hz), 3.78-3.82 (1H, m), 4.82 (1H, dd, J = 5.1, 11.4 Hz), 5.00 (1H, m), 6.52 (1H, s), 7.42 (1H, dd, J = 4.9, 8.0 Hz), 8.11 (1H, dt, J = 1.7, 8.0 Hz), 8.69 (1H, dd, J = 1.5, 4.9 Hz), 9.01 (1H, d, J = 1.9 Hz) |

Table 6

| Compound No. | $^1$H-NMR δ (ppm) |
|---|---|
| 269 | 0.85-1.11 (8H, m), 0.93 (3H, s), 126 (1H, s), 1.39-1.47 (1H, m), 1.50 (3H. s), 1.55-1.68 (5H, m), 1.87 (3H, s), 1.83-2.02 (2H, m), 2.17-2.22 (1H, m), 2.96 (1H, s), 3.79 (1H, d, J = 12.2 Hz), 3.83 (1H, d, J = 12.1 Hz), 4.85 (1H, dd, J = 4.9. 11.5 Hz), 5.04 (1H, m), 5.38 (1H, dd, J = 5.12. 11.6 Hz), 6.46 (1H, s), 7.38 (1H, dd, J = 4.8. 82 Hz), 7.69-7.80 (2H, m), 7.87 (1H, m), 8.08 (1H, dt, J = 2.2, 8.0 Hz), 822 (1H, dd, J = 1.7, 7.5 Hz), 8.67 (1H, dd, J = 1.5. 4.9 Hz), 8.98 (1H, d, J = 2.4 Hz) |

Test Example 1: Pesticidal effect against Myzus persicae

Among the compounds of formula (Ib) produced by the conventional method described above, the compounds shown in Tables 2 or 3 and pyripyropene A were tested for pesticidal effect.
A leaf disk having a diameter of 2.8 cmϕ was cut out from a cabbage grown in a pot and was placed in a 5.0 cm-Schale. Four adult aphids of Myzus persicae were released in the Schale. One day after the release of the adult aphids, the adult aphids were removed. The number of larvae at the first instar born in the leaf disk was adjusted to 10, and a test solution, which had been adjusted to a concentration of 20 ppm by the addition of a 50% aqueous acetone solution (0.05% Tween 20 added) was spread over the cabbage leaf disk. The cabbage leaf disk was then air dried. Thereafter, the Schale was lidded and was allowed to stand in a temperature-controlled room (light period 16 hr - dark period 8 hr) (25°C). Three days after the initiation of standing of the Schale, the larvae were observed for survival or death, and the death rate of larvae was calculated by the following equation.

$$\text{Death rate (\%)} = \{\text{number of dead larvae}/(\text{number of survived larvae} + \text{number of dead larvae})\} \times 100$$

As result, it was found that the death rate was not less than 80% for compounds of Nos. 218 and pyripyropene A.

Test Example 2: Pesticidal effect against Myzus persicae

Among the compounds of formula (Ib) produced by the conventional method described above, the compounds shown in Tables 2 or 3 and pyripyropene A were tested for pesticidal effect.
A leaf disk having a diameter of 2.8 cmϕ was cut out from a cabbage grown in a pot and was placed in a 5.0 cm-Schale. Four adult aphids of Myzus persicae were released in the Schale. One day after the release of the adult aphids, the adult aphids were removed. The number of larvae at the first instar born in the leaf disk was adjusted to 10, and a test solution, which had been adjusted to a concentration of 0.156 ppm by the addition of a 50% aqueous acetone solution (0.05% Tween 20 added) was spread over the cabbage leaf disk. The cabbage leaf disk was then air dried. Thereafter, the Schale was lidded and was allowed to stand in a temperature-controlled room (light period 16 hr - dark period 8 hr) (25°C). Three days after the initiation of standing, the larvae were observed for survival or death, and the death rate of larvae was calculated in the same manner as in Test Example 1.
As result, it was found that the death rate was not less than 80% for compounds of Nos 218 and 261.

Test Example 3: Pesticidal effect against Plutella xylostella

A cabbage leaf disk having a diameter of 5 cm was placed in a plastic cup. Test compounds, which had been diluted to a predetermined concentration by the addition of a 50 % aqueous acetone solution(Tween 20, 0.05% added),

were spreaded over the cabbage leaf disk by means of a spray gun, and the cabbage leaf disk was then air dried. Five larvae at the second instar of Plutella xylostella were released in the cup. The cup was then lidded, and the larvae were reared in the temperature-controlled room(25°C). Three days after the treatment, the larvae were observed for survival or death, and the death rate of the larvae was calculated in the same manner as in Test Example 1.
As a results, it was found that the death rate was not less than 80% for compounds of Nos. 218 at a concentration of 500 ppm.

Test Example 4: Pesticidal effect against Trigonotylus caelestialium

A wheat seedling was immersed for 30 seconds in a solution, in which each test compound had been diluted to a predetermined concentration by the addition of a 50 % aqueous acetone solution(Tween 20, 0.05% added). The wheat seedling was air dried, and then placed in a glass cylinder. Further, two larvae at the second instar of Trigonotylus caelestialium were released in the glass cylinder. The glass cylinder was then lidded, and the larvae were reared in the temperature-controlled room(25°C). During the test, the wheat seedling was supplid with water from the bottom of the glass cylinder. Three days after the treatment, the larvae were observed for survival or death, and the death rate of the larvae were calculated in the same manner as in Test Example 1.
As a result, it was found that the death rate was not less than 80% for compound of Nos. 218 and 261 at a concentration of 100 ppm.

## Claims

1. A compound represented by formula (Ib) or an agriculturally and horticulturally acceptable salt thereof:

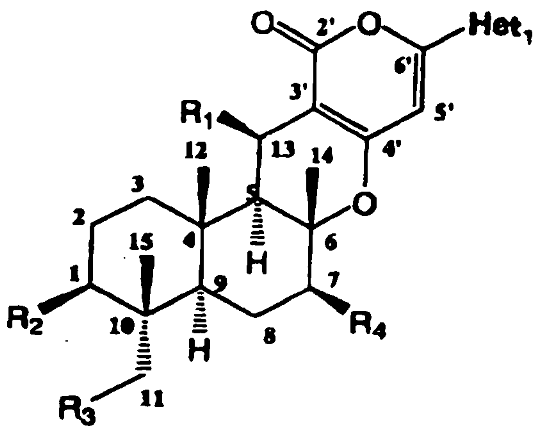

(I b)

wherein
$Het_1$ represents 3-pyridyl,
$R_1$ represents hydroxyl,
$R_2$ and $R_3$ represent cyclopropylcarbonyloxy, and
$R_4$ represents hydroxyl,
cyclopropylcarbonyloxy, or
2-cyanobenzoyloxy.

2. A composition for use as a pest control agent, comprising the compound according to claim 1 or an agriculturally and horticulturally acceptable salt thereof as active ingredient and an agriculturally and horticulturally acceptable carrier.

3. A method for controlling a pest, comprising applying an effective amount of a compound represented by formula (Ib) according to claim 1 or an agriculturally and horticulturally acceptable salt thereof to a plant or soil.

**4.** The method according to claim 3, wherein the pest is a hemipteran pest.

**5.** Non-therapeutic use of a compound represented by formula (Ib) according to claim 1 or an agriculturally and horticulturally acceptable salt thereof as a pest control agent.

**6.** Use according to claim 5, wherein the pest is a hemipteran pest.

**7.** Use of a compound represented by formuyla (Ib) according to claim 1 or an agriculturally and horticulturally acceptable salt thereof in the manufacture of a pest control agent.

**8.** Use according to claim 7, wherein the pest is a hemipteran pest.

**Patentansprüche**

**1.** Verbindung, dargestellt durch die Formel (Ib), oder ein landwirtschaftlich und gärtnerisch verträgliches Salz davon: wobei

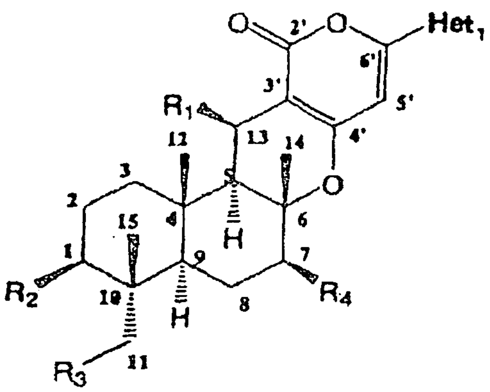

( I b )

$Het_1$ 3-Pyridyl darstellt,
$R_1$ Hydroxyl darstellt,
$R_2$ und $R_3$ Cyclopropylcarbonyloxy darstellen und
$R_4$ Hydroxyl, Cyclopropylcarbonyloxy oder 2-Cyanobenzoyloxy darstellt.

**2.** Zusammensetzung zur Verwendung als ein Schädlingsbekämpfungsmittel, umfassend die Verbindung nach Anspruch 1 oder ein landwirtschaftlich und gärtnerisch verträgliches Salz davon als aktiver Bestandteil und einen landwirtschaftlich und gärtnerisch verträglichen Träger.

**3.** Verfahren zur Kontrolle eines Schädlings, umfassend das Aufbringen einer wirksamen Menge einer durch die Formel (Ib) dargestellten Verbindung nach Anspruch 1 oder eines landwirtschaftlich und gärtnerisch verträglichen Salzes davon auf eine Pflanze oder einen Boden.

**4.** Verfahren nach Anspruch 3, wobei der Schädling ein Hemipteran-Schädling ist.

**5.** Nicht-therapeutische Verwendung einer durch die Formel (Ib) dargestellten Verbindung nach Anspruch 1 oder eines landwirtschaftlich und gärtnerisch verträglichen Salzes davon als ein Schädlingsbekämpfungsmittel.

**6.** Verwendung nach Anspruch 5, wobei der Schädling ein Hemipteran-Schädling ist.

**7.** Verwendung einer durch die Formel (Ib) dargestellten Verbindung nach Anspruch 1 oder eines landwirtschaftlich und gärtnerisch verträglichen Salzes davon in der Herstellung eines Schädlingsbekämpfungsmittels.

8. Verwendung nach Anspruch 7, wobei der Schädling ein Hemipteran-Schädling ist.

**Revendications**

1. Composé représenté par la formule (Ib) ou un sel de celui-ci acceptable sur les plans agricole et horticole :

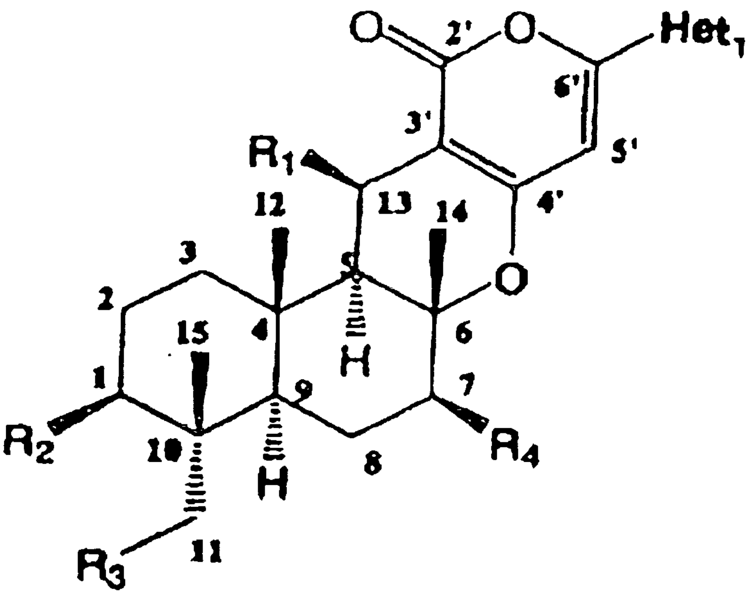

(I b)

dans laquelle
$Het_1$ représente un groupe 3-pyridyle,
$R_1$ représente un groupe hydroxyle,
$R_2$ et $R_3$ représentent un groupe cyclopropylcarbonyloxy, et
$R_4$ représente un groupe hydroxyle, cyclopropylcarbonyloxy, ou 2-cyanobenzoyloxy.

2. Composition destinée à être utilisée en tant qu'agent de lutte contre les insectes nuisibles, qui comprend le composé selon la revendication 1 ou un sel de celui-ci acceptable sur les plans agricole et horticole en tant que principe actif, et un vecteur acceptable sur les plans agricole et horticole.

3. Procédé de lutte contre un insecte nuisible, qui comprend l'application d'une quantité efficace d'un composé représenté par la formule (Ib) selon la revendication 1 ou d'un sel de celui-ci acceptable sur les plans agricole et horticole à une plante ou un sol.

4. Procédé selon la revendication 3, dans lequel l'insecte nuisible est un hémiptère.

5. Utilisation non thérapeutique d'un composé représenté par la formule (Ib) selon la revendication 1 ou d'un sel de celui-ci acceptable sur les plans agricole et horticole en tant qu'agent de lutte contre les insectes nuisibles.

6. Utilisation selon la revendication 5, l'insecte nuisible étant un hémiptère.

7. Utilisation d'un composé représenté par la formule (Ib) selon la revendication 1 ou d'un sel de celui-ci acceptable sur les plans agricole et horticole dans la fabrication d'un agent de lutte contre les insectes nuisibles.

8. Utilisation selon la revendication 7, l'insecte nuisible étant un hémiptère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2993767 B
- JP 4360895 A
- WO 9409417 A
- JP 6184158 A
- JP 8239385 A
- JP 8259569 A
- JP 8269062 A
- JP 8269063 A
- JP 8269064 A
- JP 8269065 A
- JP 8269066 A
- JP 8291164 A
- WO 2004060065 A


### Non-patent literature cited in the description


- *Journal of Antibiotics,* 1993, vol. 46 (7), 1168-9
- *Journal of Society of Synthetic Organic Chemistry, Japan,* 1998, vol. 56 (6), 478-488
- *Journal of Antibiotics,* 1997, vol. 50 (3), 229-36
- *Applied and Environmental Microbiology,* 1995, vol. 61 (12), 4429-35
- *Journal of Society of Synthetic Organic Chemistry,* 1998, vol. 56 (6), 478-488
- The Pesticide Manual. The British Crop Protection Council
- SHIBUYA INDEX. SHIBUYA INDEX RESEARCH GROUP, 2005